# EUROPEAN PATENT SPECIFICATION

(11) **EP 3 369 083 B1**
(45) Date of publication and mention of the grant of the patent: **17.02.2021**
(21) Application number: 15907170.3
(22) Date of filing: 29.10.2015
(51) Int. Cl.: G08C 19/22, H04Q 9/00, H04W 4/38, H04W 4/80, H04W 4/90, H04W 4/18, H04W 76/50, H04W 84/18, H04W 4/00, H04W 72/10, H04W 88/00

(54) **METHOD AND APPARATUS FOR FACILITATING TRANSMISSION OF A PROXIMITY HEALTH ALERT VIA A LOCAL WIRELESS NETWORK**
VERFAHREN UND VORRICHTUNG ZUR VEREINFACHTEN ÜBERTRAGUNG EINER NÄHERUNGSGESUNDHEITSWARNUNG ÜBER EIN LOKALES DRAHTLOSES NETZWERK
PROCÉDÉ ET APPAREIL POUR FACILITER LA TRANSMISSION D'UNE ALERTE DE SANTÉ DE PROXIMITÉ PAR L'INTERMÉDIAIRE D'UN RÉSEAU SANS FIL LOCAL

(43) Date of publication of application: 05.09.2018
(73) Proprietor: Nokia Technologies Oy, 02610 Espoo (FI)
(72) Inventor: ALANEN, Olli, FI-01520 Vantaa (FI); KASSLIN, Mika, FI-02770 Espoo (FI); KNECKT, Jarkko, FI-02330 Espoo (FI); MARIN, Janne, FI-02770 Espoo (FI); RANTALA, Enrico-henrik, Berkeley, CA 94708 (US)
(74) Representative: Style, Kelda Camilla Karen
(86) International application number: PCT/IB2015/058372
(87) International publication number: WO 2017/072557

(56) References cited:
- US-A1- 2002 168 958
- US-A1- 2008 166 992
- US-A1- 2008 246 629
- US-A1- 2008 246 629
- US-A1- 2009 128 320
- US-A1- 2011 001 605
- US-A1- 2011 221 591
- US-A1- 2013 109 314

## Description

### TECHNOLOGICAL FIELD

Embodiments of the present invention relate generally to a method, apparatus, and computer program product for enabling a proximity health alert, and more specifically, for facilitating the transmission of a proximity health alert from a device to other local devices upon a determination that a person is in need of local, immediate help.

### BACKGROUND

Many people at risk for various conditions continue to live relatively normal lives relying on periodic monitoring to maintain their lifestyle (e.g., going to the park, visiting relatives and friends, and the like). In the event that their health turns for the worse between monitoring sessions, emergency services may be called. However, in many instances, the person is unable to call emergency services or help is needed sooner than emergency services are able to arrive. Current methods of patient care include devices having limited mobility that are designed to monitor a patient in a hospital or medical center setting. These devices may communicate locally with external monitoring devices. For example, bedside EKG machines may trigger alarm in room where nurse is located. However, these type of devices are typically large, visible and require attention of the patient. Often the hospitals require that patients are monitored in the hospital and the patients are not allowed to leave and maintain a normal lifestyle.

Other devices are designed to provide information to a remote network and storage system, where the remote network and storage system is configured to contact the emergency personnel and/or devices in proximity. Existing devices generally require that emergency help is triggered by hand as well as remotely tracking the emergency personnel and/or devices, both of which may be problematic. Still other devices are configured to sound an alarm, ring a bell or send a message to a monitoring room in hospital. These devices do not provide information needed for first aid.

US 2008/0246629 A1 discloses a mobile device with local and remote services and applications for collecting, storing, analysing and delivering health data to medical professionals and others.

US 2009/0128320 A1 discloses a system, apparatus and method for automated emergency assistance with manual cancellation that is responsive to physiological, environmental and/or input sensors associated with an individual.

### BRIEF SUMMARY

The scope of invention is defined by the appended claims. Further embodiments of the invention are defined by the dependent claims. The embodiments which do not fall within the scope of the appended set of claims are to be interpreted as example embodiments or background information, useful only for understanding the invention.

A method, apparatus and computer program product are therefore provided according to an example embodiment of the present invention for facilitating/enabling a proximity health alert, and more specifically, for facilitating the transmission of a proximity health alert from a device to other devices nearby upon a determination that a person is in need of immediate help. In another embodiment, a method, apparatus and computer program product are provided according to an example embodiment of the present invention for requesting immediate, local help and providing relevant information necessary to, for example, render first aid or the like to a person identified as necessitating immediate, local help.

In some embodiments, a method for facilitating the transmission of a proximity health alert from a device to other local devices upon a determination that a person is in need of help and that local, immediate help is sought may be provide, the method comprising performing a condition detection process resulting in a determination of whether help is needed, causing activation of a local wireless network interface in an instance in which the performance of the condition detection process results in a determination that local help is needed, continuing to perform the condition detection process in an instance in which performance of the condition detection process results in a determination that help is not needed, and performing a help request transmission process wherein the help request transmission process comprises utilizing Bluetooth LE, scanning a first channel for at least a second device, the second device providing an address in the first channel, identifying the second device, receiving at least one indication of an available second device, the indication being an advertising packet allowing connection request, wherein the advertising packet is indicative of at least one of a connectable undirected advertising or scannable undirected advertising, causing transmission of a connection request, and upon connection, causing transmission of a help request on a second channel.

In some embodiments, in an instance in which the help request transmission process does result in identification of a helper device, the method may further comprise causing transmission of additional information to the helper device. In some embodiments, the condition detection process comprises causing reception of sensor data from sensors, causing transmission of the sensor data to server, and receiving, from the server, information indicative of the determination of whether help is needed.

In some embodiments, the condition detection process comprises causing reception of sensor data from sensors, determining, via a processor, whether help is needed. In some embodiments, the local wireless network interface is WLAN, Wi-Fi Aware, Bluetooth LE, or ZigBee.

In some embodiments, the help request transmission process comprises utilizing Wi-Fi Aware, identifying, on the basis of a geographic location, a service capable of transmitting a help request message, generating the help request message, and causing transmission of the help request message on the service. In some embodiments, wherein the help request transmission process comprises utilizing Wi-Fi Aware, identifying a service capable of transmitting a help request message, generating the help request message, and causing transmission of the help request message on the service.

In some embodiments, the help request transmission process comprises utilizing Wi-Fi Aware, activating a subscribe functionality to enable identification of one or more other devices located within communication range, and while continuing to utilize Wi-Fi Aware, receiving one or more publish notifications from at least one of the one or more other devices indicating that the at least one of the one or more other devices is within communication range. In some embodiments, the help request transmission process comprises in response to a help request transmission, receiving a request for additional information, and causing transmission of the additional information.

In some embodiments, the help request transmission process comprises prioritizing outgoing transmissions such that messages utilizing Wi-Fi Aware are caused to be transmitted prior to messages utilizing other services. In some embodiments, the help request transmission process comprises determining that the helper device is located within a predetermined distance, and causing establishment of secure connection. In some embodiments, an apparatus for facilitating the transmission of a proximity health alert from a device to other local devices upon a determination that a person is in need of help and that local, immediate help is sought may be provided, the apparatus comprising at least one processor and at least one memory including computer program code, the at least one memory and the computer program code configured to, with the processor, cause the apparatus to at least perform a condition detection process resulting in a determination of whether help is needed, cause activation of a local wireless network interface in an instance in which the performance of the condition detection process results in a determination that local help is needed, continue to perform the condition detection process in an instance in which performance of the condition detection process results in a determination that help is not needed, and perform a help request transmission process wherein the help request transmission process comprises utilizing Bluetooth LE, scanning a first channel for at least a second device, the second device providing an address in the first channel, identifying the second device, receiving at least one indication of an available second device, the indication being an advertising packet allowing connection request, wherein the advertising packet is indicative of at least one of a connectable undirected advertising or scannable undirected advertising, causing transmission of a connection request, and upon connection, causing transmission of a help request on a second channel.

In some embodiments, in an instance in which the help request transmission process does result in identification of a helper device, the at least one memory and the computer program code are further configured to, with the processor, cause the apparatus to cause transmission of additional information to the helper device. In some embodiments, the at least one memory and the computer program code are further configured to, with the processor, cause the apparatus to cause reception of sensor data from sensors, cause transmission of the sensor data to server, and receive, from the server, information indicative of the determination of whether help is needed.

In some embodiments, the at least one memory and the computer program code are further configured to, with the processor, cause the apparatus to cause reception of sensor data from sensors, determine, via a processor, whether help is needed. In some embodiments, the local wireless network interface is WLAN, Wi-Fi Aware, Bluetooth LE, or ZigBee.

In some embodiments, the at least one memory and the computer program code are further configured to, with the processor, cause the apparatus to utilizing Wi-Fi Aware, identify, on the basis of a geographic location, a service capable of transmitting a help request message, generate the help request message, and cause transmission of the help request message on the service.

In some embodiments, the at least one memory and the computer program code are further configured to, with the processor, cause the apparatus to utilize Wi-Fi Aware, identifying a service capable of transmitting a help request message, generate the help request message, and cause transmission of the help request message on the service.

In some embodiments, the at least one memory and the computer program code are further configured to, with the processor, cause the apparatus to utilize Wi-Fi Aware, activating a subscribe functionality to enable identification of one or more other devices located within communication range, and while continuing to utilize Wi-Fi Aware, receive one or more publish notifications from at least one of the one or more other devices indicating that the at least one of the one or more other devices is within communication range.

In some embodiments, the at least one memory and the computer program code are further configured to, with the processor, cause the apparatus to in response to a help request transmission, receive a request for additional information, and cause transmission of the additional information.

In some embodiments, the at least one memory and the computer program code are further configured to, with the processor, cause the apparatus to prioritize outgoing transmissions such that messages utilizing Wi-Fi Aware are caused to be transmitted prior to messages utilizing other services.

In some embodiments, the at least one memory and the computer program code are further configured to, with the processor, cause the apparatus to determine that the helper device is located within a predetermined distance, and cause establishment of secure connection.

In some embodiments, a computer program product for facilitating the transmission of a proximity health alert from a device to other local devices upon a determination that a person is in need of help and that local, immediate help is sought may be provided, the computer program product comprising at least one non-transitory computer-readable storage medium having computer-executable program code instructions stored therein, the computer-executable program code instructions comprising program code instructions for performing a condition detection process resulting in a determination of whether help is needed, causing activation of a local wireless network interface in an instance in which the performance of the condition detection process results in a determination that local help is needed, continuing to perform the condition detection process in an instance in which performance of the condition detection process results in a determination that help is not needed, performing a help request transmission process wherein the help request transmission process comprises utilizing Bluetooth LE, scanning a first channel for at least a second device, the second device providing an address in the first channel, identifying the second device, receiving at least one indication of an available second device, the indication being an advertising packet allowing connection request, wherein the advertising packet is indicative of at least one of a connectable undirected advertising or scannable undirected advertising, causing transmission of a connection request, and upon connection, causing transmission of a help request on a second channel.

In some embodiments, in an instance in which the help request transmission process does result in identification of a helper device, the computer-executable program code instructions further comprise program code instructions for causing transmission of additional information to the helper device.

In some embodiments, the computer-executable program code instructions for performing the condition detection process further comprise program code instructions for causing reception of sensor data from sensors, causing transmission of the sensor data to server, and receiving, from the server, information indicative of the determination of whether help is needed.

In some embodiments, the computer-executable program code instructions for performing the condition detection process further comprise program code instructions for causing reception of sensor data from sensors, determining, via a processor, whether help is needed. In some embodiments, the local wireless network interface is WLAN, Wi- Fi Aware, Bluetooth LE, or ZigBee. In some embodiments, the computer-executable program code instructions for performing the help request transmission process further comprise program code instructions for utilizing Wi-Fi Aware, identifying, on the basis of a geographic location, a service capable of transmitting a help request message, generating the help request message, and causing transmission of the help request message on the service.

In some embodiments, the computer-executable program code instructions for performing the help request transmission process further comprise program code instructions for utilizing Wi-Fi Aware, identifying a service capable of transmitting a help request message, generating the help request message, and causing transmission of the help request message on the service.

In some embodiments, the computer-executable program code instructions for performing the help request transmission process further comprise program code instructions utilizing Wi-Fi Aware, activating a subscribe functionality to enable identification of one or more other devices located within communication range, and while continuing to utilize Wi-Fi Aware, receiving one or more publish notifications from at least one of the one or more other devices indicating that the at least one of the one or more other devices is within communication range.

In some embodiments, the computer-executable program code instructions for performing the help request transmission process further comprise program code instructions for in response to a help request transmission, receiving a request for additional information, and causing transmission of the additional information.

In some embodiments, the computer-executable program code instructions for performing the help request transmission process further comprise program code instructions for prioritizing outgoing transmissions such that messages utilizing Wi-Fi Aware are caused to be transmitted prior to messages utilizing other services.

In some embodiments, the computer-executable program code instructions for performing the help request transmission process further comprise program code instructions for determining that the helper device is located within a predetermined distance, and causing establishment of secure connection.

In some embodiments, an apparatus may be provided for facilitating the transmission of a proximity health alert from a device to other local devices upon a determination that a person is in need of help and that local, immediate help is sought, the apparatus comprising means for performing a condition detection process resulting in a determination of whether help is needed, means for causing activation of a local wireless network interface in an instance in which the performance of the condition detection process results in a determination that local help is needed, means for continuing to perform the condition detection process in an instance in which performance of the condition detection process results in a determination that help is not needed, and performing a help request transmission process wherein the help request transmission process comprises utilizing Bluetooth LE, scanning a first channel for at least a second device, the second device providing an address in the first channel, identifying the second device, receiving at least one indication of an available second device, the indication being an advertising packet allowing connection request, wherein the advertising packet is indicative of at least one of a connectable undirected advertising or scannable undirected advertising, causing transmission of a connection request, and upon connection, causing transmission of a help request on a second channel.

In some embodiments, in an instance in which the help request transmission process does result in identification of a helper device, the apparatus further comprising means for causing transmission of additional information to the helper device.

In some embodiments, the means for performing the condition detection process comprises means for causing reception of sensor data from sensors, means for causing transmission of the sensor data to server, and means for receiving, from the server, information indicative of the determination of whether help is needed.

In some embodiments, the means for performing the condition detection process comprises means for causing reception of sensor data from sensors, means for determining, via a processor, whether help is needed. In some embodiments, the local wireless network interface is WLAN, Wi-Fi Aware, Bluetooth LE, or ZigBee.

In some embodiments, the means for performing the help request transmission process comprises means for utilizing Wi-Fi Aware, identifying, on the basis of a geographic location, a service capable of transmitting a help request message, means for generating the help request message, and means for causing transmission of the help request message on the service.

In some embodiments, the means for performing the help request transmission process comprises means for utilizing Wi-Fi Aware, identifying a service capable of transmitting a help request message, means for generating the help request message, and means for causing transmission of the help request message on the service.

In some embodiments, the means for performing the help request transmission process comprises means for utilizing Wi-Fi Aware, activating a subscribe functionality to enable identification of one or more other devices located within communication range, and while continuing to utilize Wi-Fi Aware, means for receiving one or more publish notifications from at least one of the one or more other devices indicating that the at least one of the one or more other devices is within communication range.

In some embodiments, the means for performing the help request transmission process comprises means for, in response to a help request transmission, receiving a request for additional information, and means for causing transmission of the additional information.

In some embodiments, the means for performing the help request transmission process comprises means for prioritizing outgoing transmissions such that messages utilizing Wi-Fi Aware are caused to be transmitted prior to messages utilizing other services. In some embodiments, the means for performing the help request transmission process comprises means for determining that the helper device is located within a predetermined distance, and means for causing establishment of secure connection.

### BRIEF DESCRIPTION OF THE DRAWINGS

Having thus described embodiments of the invention in general terms, reference will now be made to the accompanying drawings, which are not necessarily drawn to scale, and wherein:
Figure 1 is block diagram of a system that may be specifically configured in accordance with an example embodiment of the present invention;
Figure 2 is a block diagram of an apparatus that may be specifically configured in accordance with an example embodiment of the present invention;
Figures 3A-3E are example structural components of NAN service discovery frames in accordance with an embodiment of the present invention;
Figure 4 is an example flowchart illustrating a method for enabling a proximity health alert in accordance with an embodiment of the present invention;
Figure 5 is an example flowchart illustrating a method for performing a condition detection process in accordance with an embodiment of the present invention;
Figures 6A-6D are example flowcharts illustrating various methods for performing a health request transmission process in accordance with an embodiment of the present invention;
Figure 7 is an example flowchart illustrating a method for performing an information transfer process in accordance with an embodiment of the present invention; and
Figure 8 is an example flowchart illustrating a method for supplementing existing emergency service architecture with immediate local help identified via the proximity alert processes shown above, in accordance with an embodiment of the present invention.

### DETAILED DESCRIPTION

Some example embodiments will now be described more fully hereinafter with reference to the accompanying drawings. Like reference numerals refer to like elements throughout. The terms "data," "content," "information," and similar terms may be used interchangeably, according to some example embodiments, to refer to data capable of being transmitted, received, operated on, and/or stored. Moreover, the term "exemplary", as may be used herein, is not provided to convey any qualitative assessment, but instead merely to convey an illustration of an example.

As used herein, the term "circuitry" refers to all of the following: (a) hardware-only circuit implementations (such as implementations in only analog and/or digital circuitry); (b) to combinations of circuits and software (and/or firmware), such as (as applicable): (i) to a combination of processor(s) or (ii) to portions of processor(s)/software (including digital signal processor(s)), software, and memory(ies) that work together to cause an apparatus, such as a mobile phone or server, to perform various functions); and (c) to circuits, such as a microprocessor(s) or a portion of a microprocessor(s), that require software or firmware for operation, even if the software or firmware is not physically present.

This definition of "circuitry" applies to all uses of this term in this application, including in any claims. As a further example, as used in this application, the term 'circuitry' would also cover an implementation of merely a processor (or multiple processors) or portion of a processor and its (or their) accompanying software and/or firmware. The term 'circuitry' would also cover, for example and if applicable to the particular claim element, a baseband integrated circuit or application specific integrated circuit for a mobile phone or a similar integrated circuit in a server, a cellular network device, or other network device.

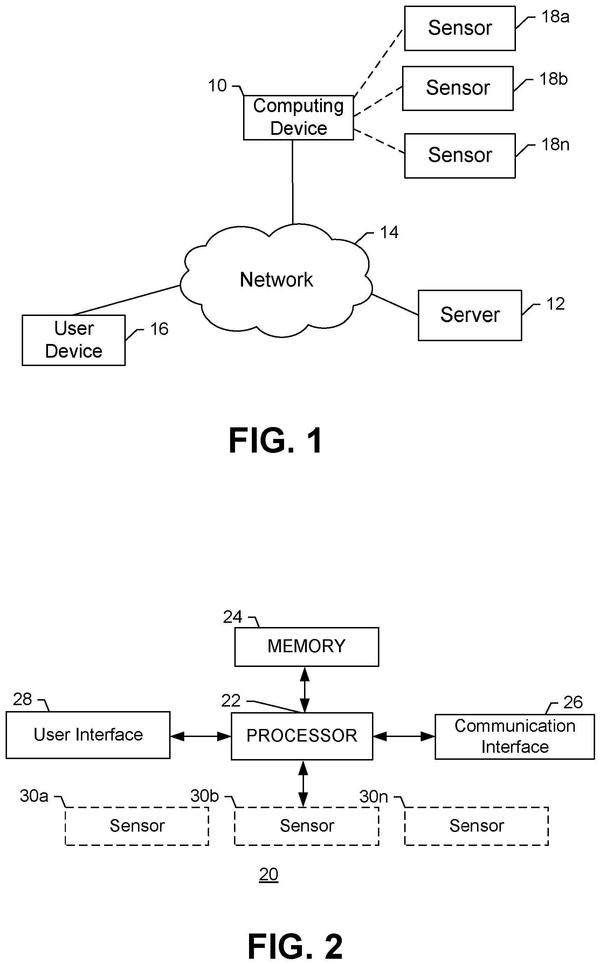

Referring now of Figure 1, a system that supports communication, either wirelessly or via a wireline, between a computing device 10, sensors 18a, 18b, and 18n, user device 16, and server 12 or other network entity (hereinafter generically referenced as a "server") is illustrated. As shown, the computing device 10, sensors, 18a-n, user device 16 and the server 12 may be in communication via a network 14, such as a wide area network, such as a cellular network or the Internet, or a personal or local area network such as a personal or local wireless network (e.g. a variant of Wi-Fi or Bluetooth (BT) such as Wi-Fi infrastructure, Wi-Fi Aware or Bluetooth Low Energy (BLE)). However, the computing device 10, sensors, 18a-n, user device 16 and the server 12 may be in communication in other manners, such as via direct communications between the computing device and the server. The computing device 10, sensors, 18a-n, user device 16 and the server 12 may be hereinafter described as mobile devices and/or mobile terminals, but may be either mobile or fixed in the various embodiments.

The computing device 10 and user device 16 may be embodied by a number of different devices including mobile computing devices, such as a personal digital assistant (PDA), mobile telephone, smartphone, laptop computer, tablet computer, or any combination of the aforementioned, and other types of voice and text communications systems. Alternatively, the computing device 10 may be a fixed computing device, such as a personal computer, a computer workstation or the like. The sensors 18a-18n may be any object or device configured to detect events or changes in its environment, and then provide a corresponding output. Here, the sensors 18a-18n may detect a particular condition related to a patient (e.g., heartbeat, temperature, or the like) or particular events or changes in a condition of a patient (e.g., fever, oxygen levels in the blood, falling, or the like). The server 12 may also be embodied by a computing device and, in one embodiment, is embodied by a web server. Additionally, while the system of Figure 1 depicts a single server, the server may be comprised of a plurality of servers which may collaborate to support browsing activity conducted by the computing device.

Regardless of the type of device that embodies the computing device 10, the computing device 10 and/or user device 16 may include or be associated with an apparatus 20 as shown in Figure 2. In this regard, the apparatus may include or otherwise be in communication with a processor 22, a memory device 24, a communication interface 26 and a user interface 28. Additionally, in some embodiments, the apparatus may also include one or more sensor 30a, 30b, and 30n. As such, in some embodiments, although devices or elements are shown as being in communication with each other, hereinafter such devices or elements should be considered to be capable of being embodied within the same device or element and thus, devices or elements shown in communication should be understood to alternatively be portions of the same device or element.

In some embodiments, the processor 22 (and/or co-processors or any other processing circuitry assisting or otherwise associated with the processor) may be in communication with the memory device 24 via a bus for passing information among components of the apparatus. The memory device may include, for example, one or more volatile and/or non-volatile memories. In other words, for example, the memory device may be an electronic storage device (e.g., a computer readable storage medium) comprising gates configured to store data (e.g., bits) that may be retrievable by a machine (e.g., a computing device like the processor). The memory device may be configured to store information, data, content, applications, instructions, or the like for enabling the apparatus 20 to carry out various functions in accordance with an example embodiment of the present invention. For example, the memory device could be configured to buffer input data for processing by the processor. Additionally or alternatively, the memory device could be configured to store instructions for execution by the processor.

As noted above, the apparatus 20 may be embodied by a computing device 10 configured to employ an example embodiment of the present invention. However, in some embodiments, the apparatus may be embodied as a chip or chip set. In other words, the apparatus may comprise one or more physical packages (e.g., chips) including materials, components and/or wires on a structural assembly (e.g., a baseboard). The structural assembly may provide physical strength, conservation of size, and/or limitation of electrical interaction for component circuitry included thereon. The apparatus may therefore, in some cases, be configured to implement an embodiment of the present invention on a single chip or as a single "system on a chip." As such, in some cases, a chip or chipset may constitute means for performing one or more operations for providing the functionalities described herein.

The processor 22 may be embodied in a number of different ways. For example, the processor may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processor may include one or more processing cores configured to perform independently. A multi-core processor may enable multiprocessing within a single physical package. Additionally or alternatively, the processor may include one or more processors configured in tandem via the bus to enable independent execution of instructions, pipelining and/or multithreading.

In an example embodiment, the processor 22 may be configured to execute instructions stored in the memory device 24 or otherwise accessible to the processor. Alternatively or additionally, the processor may be configured to execute hard coded functionality. As such, whether configured by hardware or software methods, or by a combination thereof, the processor may represent an entity (e.g., physically embodied in circuitry) capable of performing operations according to an embodiment of the present invention while configured accordingly. Thus, for example, when the processor is embodied as an ASIC, FPGA or the like, the processor may be specifically configured hardware for conducting the operations described herein. Alternatively, as another example, when the processor is embodied as an executor of software instructions, the instructions may specifically configure the processor to perform the algorithms and/or operations described herein when the instructions are executed. However, in some cases, the processor may be a processor of a specific device (e.g., a head mounted display) configured to employ an embodiment of the present invention by further configuration of the processor by instructions for performing the algorithms and/or operations described herein. The processor may include, among other things, a clock, an arithmetic logic unit (ALU) and logic gates configured to support operation of the processor. In one embodiment, the processor may also include user interface circuitry configured to control at least some functions of one or more elements of the user interface 28.

Meanwhile, the communication interface 26 may be any means such as a device or circuitry embodied in either hardware or a combination of hardware and software that is configured to receive and/or transmit data between the computing device 10 and a server 12. In this regard, the communication interface 26 may include, for example, an antenna (or multiple antennas) and supporting hardware and/or software for enabling communications wirelessly. Additionally or alternatively, the communication interface may include the circuitry for interacting with the antenna(s) to cause transmission of signals via the antenna(s) or to handle receipt of signals received via the antenna(s). For example, the communications interface may be configured to communicate wirelessly with the head mounted displays 10, such as via Wi-Fi, Bluetooth or other wireless communications techniques. In some instances, the communication interface may alternatively or also support wired communication. As such, for example, the communication interface may include a communication modem and/or other hardware/software for supporting communication via cable, digital subscriber line (DSL), universal serial bus (USB) or other mechanisms. For example, the communication interface may be configured to communicate via wired communication with other components of the computing device.

The user interface 28 may be in communication with the processor 22, such as the user interface circuitry, to receive an indication of a user input and/or to provide an audible, visual, mechanical, or other output to a user. As such, the user interface may include, for example, a keyboard, a mouse, a joystick, a display, a touch screen display, a microphone, a speaker, and/or other input/output mechanisms. In some embodiments, a display may refer to display on a screen, on a wall, on glasses (e.g., near-eye-display), in the air, etc. The user interface may also be in communication with the memory 24 and/or the communication interface 26, such as via a bus.

In an example embodiment of the present invention, a user may utilize a system, including a computing device, such as a mobile telephone, tablet, laptop, or the like to facilitate the transmission of a proximity health alert from the computing device to other local devices upon a determination that a person is in need of local, immediate help.

As described above, the network 14 shown in Figure 1 may be a personal or local wireless network which may be based on a wireless industry standard such as Wi-Fi Aware ™ or Bluetooth Low Energy (BLE). Wi-Fi Aware ™ is a Wi-Fi Alliance certification program configured to ensure interoperability of devices that implement features specified in Wi-Fi Neighbor Awareness Networking (NAN) technical specification that has been developed in Wi-Fi Alliance. The NAN provides means for discovery of services, information, etc. that can be run continuously in the background of a device without significant effect on standby time of the device. The NAN may make all or near all of the NAN capable devices that have the NAN stack available at same time in same channel for discovery. Common time periods are called NAN discovery windows and happen approximately every .5 seconds. NAN devices exchange NAN service discovery frames during the NAN discovery windows and each discovery frame may contain one or more publish, subscribe or follow-up messages.

Publish, as used herein, is a mechanism to make a service/application discoverable in a device and subscribe, as used herein, is a mechanism to look for services/applications available in devices close by (e.g., within a predefined distance, within the wireless network capabilities of the device, or the like). Publish may be run in either solicited or unsolicited mode, and, in some embodiments, publish may be run simultaneously in both solicited and unsolicited mode. When run in the solicited mode, publish may transmit a publish message only upon receiving a subscribe message that meets the publish conditions. When run in the unsolicited mode, publish may transmit publish messages independent of subscribe messages. Subscribe may be run in active and/or passive mode. In the active mode, subscribe messages may be transmitted to trigger transmission of publish messages in devices in which the service/application that one is looking for has been made discoverable. In the passive mode no subscribe messages are transmitted but instead, discovery may be performed based on received publish messages. Follow-up, as used herein, is a mechanism for at least two service/application layers of at least two peer NAN devices to communicate with each other upon successful NAN service discovery.

For example, a NAN device that has subscribed for a service and discovered a NAN device that publishes for the service, may transmit a follow-up message related to the service and directed to the publishing device. The follow-up mechanism may be used, for example, by a subscribing device to request more information on the published service. A NAN Discovery Engine may not, however, process the follow-up frames but, instead, pass them to the service layer as long as the values of the Service ID (48-bit service identifier) and the Instance ID fields of the received follow-up frame match with an active publish/subscribe instance in the device.

Service name, as used herein, may be used as the label of a service/application that is either available (e.g., publishing) for others to use or that is sought (e.g., subscribing) from other devices. The Wi-Fi Aware stack may generate a 48-bit service identifier from the service name by using a specified hash function and that service identifier is used in publish/subscribe/follow-up messages to indicate the service to which the message relates. In some embodiments, reverse domain name notation may be used with all or near all of the services. Service names that start with the symbol "org.wi-fi" are reserved for services defined by the Wi-Fi Alliance.

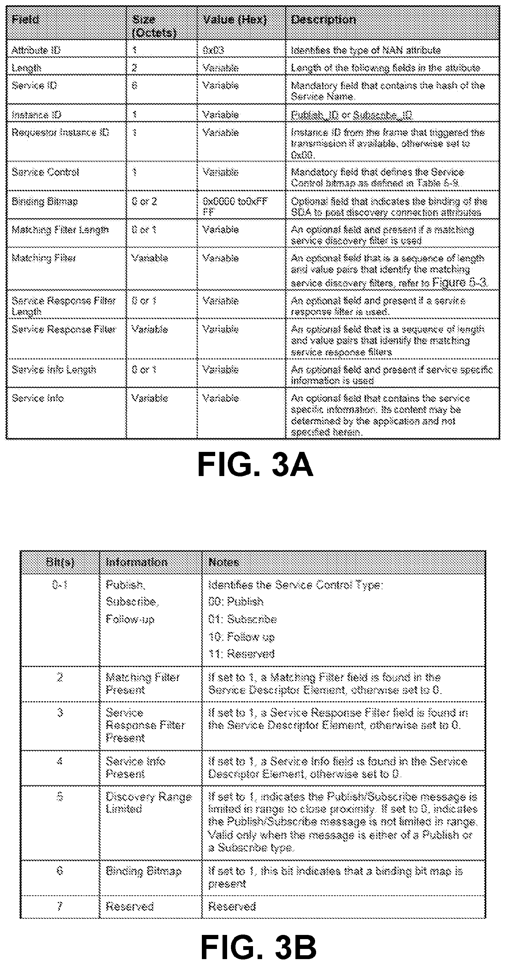

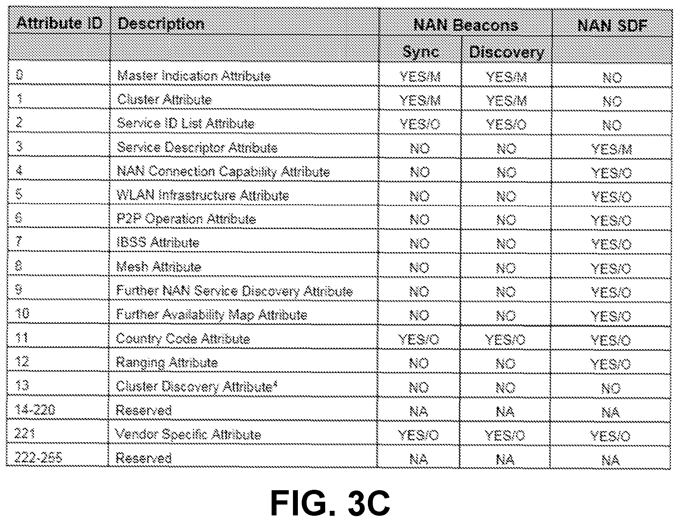

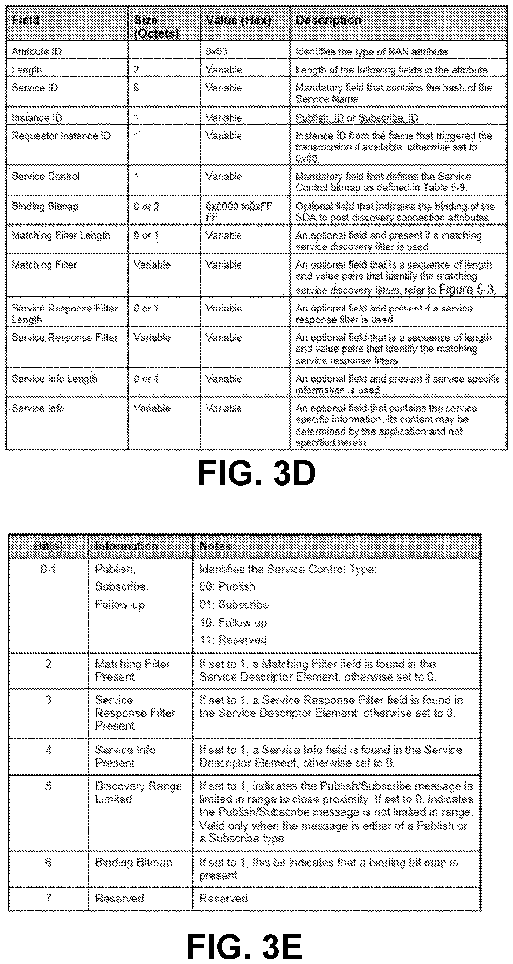

A NAN service discovery frame is a NAN specific Public Action frame that has the structure illustrated in Figure 3A. Each discovery frame contains at least one NAN Attribute (as shown in Figure 3B) that is a Service Descriptor Attribute (as shown in Figure 3C). The Service Descriptor Attribute is used to carry publish, subscribe and follow-up messages and the Service Control field (Figure 3D) contains information e.g. on the message type. The Matching Filter field may be used to indicate further conditions beyond the Service ID for discovery and the Service Info field may be used to carry service specific information to the service/application layer of the peer device. The Service ID field may indicate the service to which the publish/subscribe/follow-up message relates. The Instance ID field may carry an identifier of the publish/subscribe instance that generated the transmitted message and the Requestor Instance ID may be used to indicate the publish/subscribe instance in the peer device that triggered transmission of the message. Requestor Instance ID field may be set to '0' when the message is transmitted autonomously without a trigger message from a peer device

Bluetooth Low Energy (LE) (i.e. Bluetooth Smart) is a Bluetooth version configured for low power operations. A Bluetooth LE device (a device configured to support Bluetooth LE) may be configured to be discoverable and to offer services for other devices close by using advertising to indicate presence and availability of itself and/or its' services. An advertising device may transmit advertising channel packets in dedicated advertising channels and may listen to and respond to responses triggered by the advertising packets. A device may be also acting as a scanner or as an initiator. When scanning, a device may look for advertising devices and may request further information from detected advertising devices. A device may operate as an initiator when establishing or attempting to establish a connection with another device. An initiator may request a connection with an advertising device upon receiving an advertising packet which allows a connection request.

An advertising device may utilize any of at least four different types of advertising schemes: a) connectable undirected advertising, b) connectable directed advertising, c) scannable undirected advertising, and d) non-connectable undirected advertising. The connectable undirected advertising scheme may be configured such that an advertising device may transmit advertising packets, the advertising packets indicative of availability and allowing other devices to request more information and/or connection.

When a device uses connectable undirected advertising, the device may be configured to allow other devices to request more information (scanner) and/or to request connection (initiator). Upon receiving a scan request packet from any device (scanner) after a transmitted advertising packet, the advertising device transmits a scan response with more information about, for example, the device and its services. Upon receiving a connection request packet from any device (initiator) after a transmitted advertising packet, the advertising device may move transmission to the data channel for connect establishment per the information received in the connection request packet.

When a device uses connectable directed advertising, the device may be configured to allow only the device indicated in the transmitted advertising packets to request a connection. Other devices may not request further information or connection.

When a device uses scannable undirected advertising, the device may be configured to allow any other devices to request further information. The device may be further configured to not allow connection requests or connections. Upon receiving a scan request packet from any device (scanner) after a transmitted advertising packet, the advertising device transmits a scan response with more information about, for example, the device and its services. Connection request packets may be ignored.

When a device uses non-connectable undirected advertising, the device may be configured to not allow or refuse requests for further information or for connection and/or ignore any such requests.

Each advertising packet, except those transmitted with connectable directed advertising, may contain advertising data that typically may be determined by the host of the advertising device. In the connectable directed advertising scheme, the advertising packet may not contain any such data but, instead, the packet may carry only the address of the device to which the packet is targeted as well as the advertiser's own address which is present in all the advertising packets.

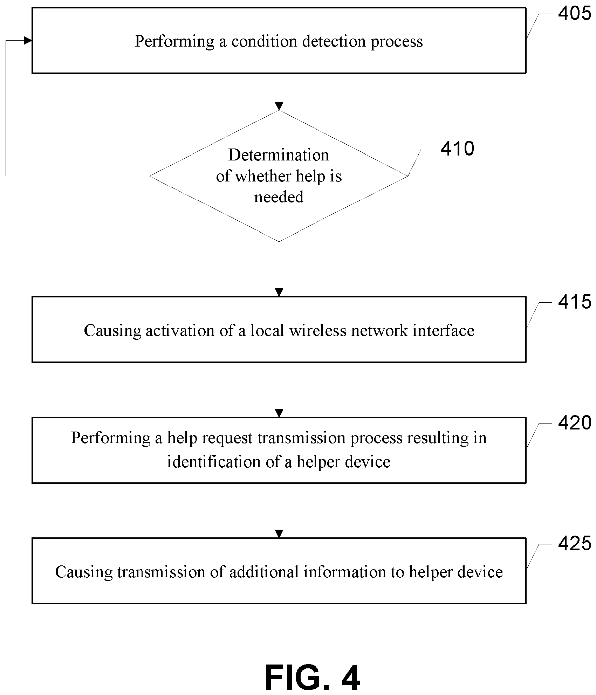

Figures 4, 5, 6A-6D, 7, and 8 illustrate example flowcharts of the example operations performed by a method, apparatus and computer program product in accordance with an embodiment of the present invention. It will be understood that each block of the flowcharts, and combinations of blocks in the flowcharts, may be implemented by various means, such as hardware, firmware, processor, circuitry and/or other device associated with execution of software including one or more computer program instructions. For example, one or more of the procedures described above may be embodied by computer program instructions. In this regard, the computer program instructions which embody the procedures described above may be stored by a memory 24 of an apparatus employing an embodiment of the present invention and executed by a processor 22 in the apparatus. As will be appreciated, any such computer program instructions may be loaded onto a computer or other programmable apparatus (e.g., hardware) to produce a machine, such that the resulting computer or other programmable apparatus provides for implementation of the functions specified in the flowchart block(s). These computer program instructions may also be stored in a non-transitory computer-readable storage memory that may direct a computer or other programmable apparatus to function in a particular manner, such that the instructions stored in the computer-readable storage memory produce an article of manufacture, the execution of which implements the function specified in the flowchart block(s). The computer program instructions may also be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowchart block(s). As such, the operations of Figures 4, 5, 6A-6D, 7, and 8, when executed, convert a computer or processing circuitry into a particular machine configured to perform an example embodiment of the present invention. Accordingly, the operations of Figures 4, 5, 6A-6D, 7, and 8 define an algorithm for configuring a computer or processing to perform an example embodiment. In some cases, a general purpose computer may be provided with an instance of the processor which performs the algorithms of Figures 4, 5, 6A-6D, 7, and 8 to transform the general purpose computer into a particular machine configured to perform an example embodiment.

Accordingly, blocks of the flowchart support combinations of means for performing the specified functions and combinations of operations for performing the specified functions. It will also be understood that one or more blocks of the flowcharts, and combinations of blocks in the flowcharts, can be implemented by special purpose hardware-based computer systems which perform the specified functions, or combinations of special purpose hardware and computer instructions.

In some embodiments, certain ones of the operations herein may be modified or further amplified as described below. Moreover, in some embodiments additional optional operations may also be included as shown by the blocks having a dashed outline in Figures 4, 5, 6A-6D, 7, and 8. It should be appreciated that each of the modifications, optional additions or amplifications below may be included with the operations above either alone or in combination with any others among the features described herein.

### PROXIMITY HEALTH ALERT

Embodiments of the present invention relate generally to a method, apparatus, and computer program product for facilitating/enabling a proximity health alert, and more specifically, for enabling a device or a set of devices that, for example, configured to monitor a person's state or health condition, to announce, via wireless means, locally, that the person requires immediate/near immediate help. While traditional methods exist (e.g., calling 911 and waiting for an ambulance), embodiments of the present invention complement existing infrastructure by providing means to attract immediate or near immediate local help. That is, people who are capable of (or, in some instances, responsible for) providing assistance (e.g., medical personnel) may carry means with which they are able to detect the proximity health alert and identify persons close by that require help.

Some embodiments provided herein provide mechanisms facilitating the categorization of local announcements based on a type of the help needed, which may be used to attract people with specific skills that are locally available to help the person in need.

Other embodiments provided herein provide mechanisms with which the user device, operated or possessed by a person willing and/or able to provide assistance, may connect wirelessly to the computing device(s), operating or possessed by a person in need of assistance that has made the local announcement. These mechanisms are activated only upon the determination that the user device is close enough to the computing device and that the computing device allows for the user device to receive or access more information, the situation, the reasons for the announcement, medical history, etc. that benefit the immediate or near immediate assistance, for example, before more help (e.g., an ambulance) is available.

Figure 4 is an example flowchart illustrating a method for enabling a proximity health alert in accordance with an embodiment of the present invention. It should be appreciated that the operations of Figure 4 may be performed by a cellular phone, or a "smart" phone. In some embodiments, the operations of Figure 4 are not limited to any particular network (e.g., cellular systems). For example, networking solutions such as a wireless local area network (WLAN) or a wireless personal area network (WPAN) (e.g., Wi-Fi Aware or over Bluetooth LE) may similarly permit the facilitation of the proximity health alert process described in Figure 4. Moreover, the operations of Figure 4 may be performed by any other computing device, such as a laptop, tablet, or desktop computer or the like (collectively referred to as a patient device or computing device).

In an exemplary embodiment of the present invention, a user may utilize a system, including, as described above, a computing device, such as a smart phone or the like to perform a condition detection process resulting in a determination of whether help is needed.

For example, the heart rate or oxygen saturation of a user may be monitored by one or more sensors and based on sensor data received from any of the one or more sensors, a determination may be made as to whether help is needed. Figure 4 further describes the condition detection process in greater detail.

As such, as shown in block 405 of Figure 4, an apparatus, such as apparatus 20 embodied by the computing device 10 or user device 16, may be configured to cause performing of a condition detection process. The apparatus embodied by user device 16 therefore includes means, such as the processor 22, the communication interface 26 or the like, for performing of a condition detection process. During the condition detection process, or as a result of the condition detection process, a determination may be made as to whether help is needed and whether immediate, local help is sought. As such, as shown in block 410 of Figure 4, an apparatus, such as apparatus 20 embodied by the computing device 10 or user device 16, may be configured to cause a determination as to whether help is needed. The apparatus may therefore include means, such as the processor 22, the communication interface 26 or the like, for determining whether help is needed.

In the event that a determination is made that help is needed, a local wireless network interface may be activated whereas, in the event that a determination is made that help is not needed, the condition detection process may be continued.

As such, as shown in block 415 of Figure 4, an apparatus, such as apparatus 20 embodied by computing device 10 or the user device 16, may be configured to cause activation of a local wireless network interface in an instance in which the performance of the condition detection process results in a determination that help is needed. The apparatus, such as the processor 22, the communication interface 26 or the like, may therefore include means for causing activation of a local (and/or personal) wireless network interface in an instance in which the performance of the condition detection process results in a determination that help is needed.

In an instance in which it is determined that help is not needed, the condition detection process made be continued and/or resumed. As such, an apparatus, such as apparatus 20 may be configured to cause the continuance of the condition detection process in an instance in which performance of the condition detection process results in a determination that help is not needed. The apparatus may therefore include means, such as the processor 22, the communication interface 26 or the like, for continuing to perform the condition detection process in an instance in which performance of the condition detection process results in a determination that help is not needed.

Subsequent to the activation of the local wireless network interface, a help request may be transmitted. As such, as shown in block 420 of Figure 4, an apparatus, such as apparatus 20 embodied by computing device 10 or the user device 16, may be configured to perform a help request transmission process. The apparatus may therefore include means, such as the processor 22, the communication interface 26 or the like, for performing a help request transmission process. In some embodiments, the performance of the help request transmission process may result in the identification of a helper device. For example, detection of a helper device may include the help request transmission, which is then received by the helper device who may then decide to respond (or, in some embodiments, not respond), and subsequently transmit their response. Once the response is transmitted, the response may be received by the apparatus, who may then identify the helper device.

In those embodiments in which a helper device is identified, additional information may be transmitted. As such, as shown in block 425 of Figure 4, an apparatus, such as apparatus 20 embodied by computing device 10 or the user device 16, may be configured to cause transmission of additional information to helper device. The apparatus may therefore include means, such as the processor 22, the communication interface 26 or the like, for causing transmission of additional information to helper device.

### CONDITION DETECTION PROCESS

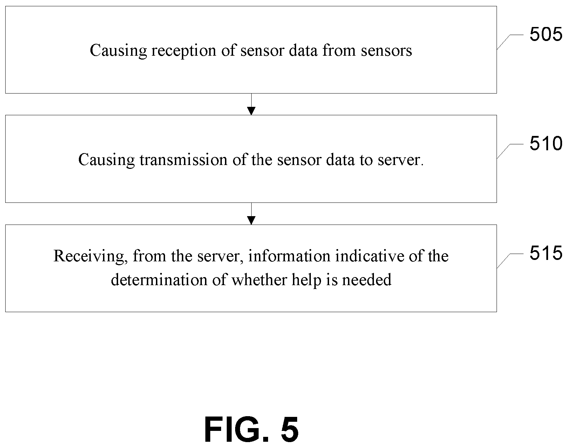

Figure 5 is an example flowchart illustrating a method for performing a condition detection process in accordance with an embodiment of the present invention. It should be appreciated that the operations of Figure 5 may be performed by a smart phone, though in some embodiments, are not limited to cellular systems. For example, non-cellular solutions such as a wireless local area network (WLAN) may similarly permit the performance of the condition detection process. Moreover, the operations of Figure 5 may be performed by any other computing device, such as a laptop, tablet, or desktop computer or the like utilizing any type of network.

In an exemplary embodiment of the present invention, a user may utilize a system, including, as described above, a computing device, such as a smart phone or the like to perform the condition detection process that utilizes sensor data received or accessed from one or more sensors to determine whether help is needed. The condition detection process may, for example, include the utilization of sensor data related the heart rate of the user, such that sensor data indicating that the heart rate is not within a normal or predefined range triggers a determination that help is needed. Other instances of sensor data may include the oxygen saturation, temperature, or the like. In other instances, sensor data may identify if the user is experiencing epileptic or other uncontrollable symptoms, has experienced a big collision, large acceleration, fall, decreased mobility/range of motion. In one embodiment, the user may be suffering from, for example, Alzheimer's disease and not know where he/she is currently. Here, sensor data may be indicative of a local alarm message resulting from, for example, a press of a help button indicating that he/she needs help.

The condition detection process and a subsequent determination of whether to activate a help request transmission process (e.g., when help is needed) may be done locally in the device, in a cloud service or both. That is, the device and the cloud service may have a role in the decision making. In some embodiments, the system is configured to operate according to each principle. While, in some embodiments, the determination may be made by the cloud service based on the information from the device, additional embodiments may be configured such that the device is enabled to perform the determination, for example, to ensure that help will be available even if connectivity between the device and the cloud service is unavailable and/or not of high quality.

As such, as shown in block 505 of Figure 5, an apparatus, such as apparatus 20 embodied by computing device 10 or the user device 16, may be configured to cause reception of sensor data from sensors. The apparatus may therefore include means, such as the processor 22, the communication interface 26 or the like, for causing reception of sensor data from sensors.

In an embodiment, in which the determination of whether help is needed is done by a cloud service or otherwise, non-locally, such as by server 12, the sensor data may be transmitted to the apparatus that is configured to make the determination and/or transmit the received sensor data to server. As such, as shown in block 510 of Figure 5, an apparatus, such as apparatus 20 may be configured to cause transmission of the sensor data to server, for example server 12 as shown in Figure 1. The apparatus may therefore include means, such as the processor 22, the communication interface 26 or the like, for causing transmission of the sensor data to server, such as for example server 12. In those embodiments in which the determination may be made locally, the apparatus may be configured to store sensor data, for example, in memory 24.

Where sensor data is transmitted to, for example, server 12 or any other service or apparatus configured to make the determination, after the determination is made, the results of the determination may then be transmitted back to the apparatus. As such, as shown in block 515 of Figure 5, an apparatus, such as apparatus 20 embodied by computing device 10 or the user device 16, may be configured to cause reception, from the server, information indicative of the determination of whether help is needed. The apparatus may therefore include means, such as the processor 22, the communication interface 26 or the like, for receiving, from the server, information indicative of the determination of whether help is needed.

### A CALL FOR HELP

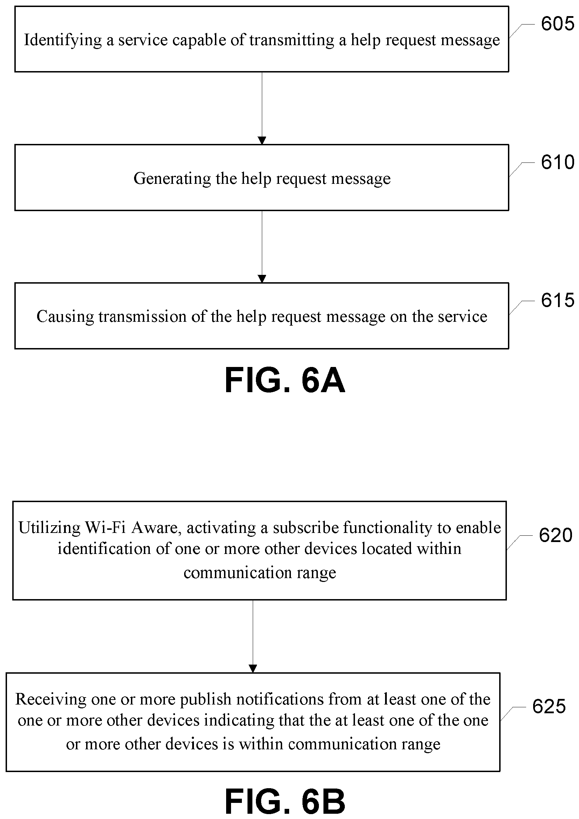

Based on the detection of a particular condition in the condition detection process, a determination that help is needed may be made and/or a help request process may be initiated and/or activated. Figures 6A-6D are example flowcharts illustrating various methods for performing a health request transmission process in accordance with an embodiment of the present invention. It should be appreciated that the operations of Figures 6A-6D may be performed by a smart phone. In some embodiments, the operations of Figures 6A-6D are not limited to any particular network (e.g., cellular systems). For example, non-cellular solutions such as a wireless local area network (WLAN) or a wireless personal area network (WPAN) (e.g., Wi-Fi Aware or over Bluetooth LE) may similarly permit the performance of the health request transmission process described in Figures 6A-6D. Moreover, the operations of Figures 6A-6D may be performed by any other computing device, such as a laptop, tablet, or desktop computer or the like.

In an exemplary embodiment of the present invention, upon activating the help request transmission process, for example, either upon receiving a request from the cloud service or upon internal decision to activate the process, the computing device may initiate or cause operation of at least one local wireless interface of one or more available local wireless interfaces configured for use in the device. The local wireless interface may be initiated in a mode that is designed to attract local helpers. That is, the local wireless interface may use the Wi-Fi Aware (i.e. Neighbor Awareness Networking (NAN)) or Bluetooth Low Energy (Bluetooth LE) (i.e. Bluetooth Smart) technology, or any other similar local or personal area wireless technology (e.g., ZigBee).

As described above, in some embodiments, the local wireless interface may utilize the Wi-Fi Aware (i.e. NAN) technology. In some embodiments, a service name may be used as a label indicating need of local help in general. As shown in block 605 of Figure 6A, an apparatus, such as apparatus 20 embodied by computing device 10 or the user device 16, may be configured to identify a service capable of transmitting a help request message. The apparatus may therefore include means, such as the processor 22, the communication interface 26 or the like, for identifying a service capable of transmitting a help request message. In some embodiments, a service name that, for example, may be known to represent a request or need for local help may be determined. The name can be allocated, for example, on country-by-country basis (e.g., fi.apu.paikallis in Finland) or other geographical basis. Additionally or alternatively, the service name may be determined on an organizational basis. That is, the service name may be determined based on an organization (e.g., International Red Cross) that may possess, enable or facilitate use of a specific service name independent of location (e.g., org.irc.help.local). Such an approach may facilitate attraction of local help more effectively than if the service name was specific and/or provided too much information about the case and required help.

Subsequent to identifying a service and/or service name, as shown in block 605 of Figure 6A, an apparatus, such as apparatus 20 embodied by computing device 10 or the user device 16, may be configured to generate a help request message. The apparatus may therefore include means, such as the processor 22, the communication interface 26 or the like, for generating a help request message. Once generated, the help request message may be transmitted. As such, as shown in block 605 of Figure 6A, an apparatus, such as apparatus 20 embodied by computing device 10 or the user device 16, may be configured to cause transmission of the help request message on the service. The apparatus may therefore include means, such as the processor 22, the communication interface 26 or the like, for causing transmission of the help request message on the service.

Additionally or alternatively, in some embodiments, the service name may be utilized to provide information as to a type of the help needed. For example, in some embodiments, in an instance in which the service name provides more information than just the need for help, only persons who are experts on in the request field of required help and are able offer required help may be made aware of the situation/request. However, in many instances, any help from qualified persons is better than no help at all. As such, in some embodiments, the service name may represent a type of requested help. For example, a service name such as org.irc.help.local.heart may be utilized to indicate, for example, that the person in need of help (a patient) has heart problems and medical experts capable of helping with the situation are needed.

In some embodiments, both publish and subscribe may be used to indicate the need of help and/or to attract attention of people close by (e.g., within the local wireless network range) that are capable and/or willing to provide help. As such, as shown in block 620 of Figure 6B, an apparatus, such as apparatus 20 embodied by computing device 10 or the user device 16, may be configured to, utilizing Wi-Fi Aware, activate a subscribe functionality to enable identification of one or more other devices located within communication range. The apparatus may therefore include means, such as the processor 22, the communication interface 26 or the like, for, utilizing Wi-Fi Aware, activating a subscribe functionality to enable identification of one or more other devices located within communication range. Next, as shown in block 625 of Figure 6B, an apparatus, such as apparatus 20 embodied by computing device 10 or the user device 16, may be configured to, while continuing to utilize Wi-Fi Aware, receive one or more publish notifications from at least one of the one or more other devices indicating that the at least one of the one or more other devices is within communication range. The apparatus may therefore include means, such as the processor 22, the communication interface 26 or the like, for, while continuing to utilize Wi-Fi Aware, receiving one or more publish notifications from at least one of the one or more other devices indicating that the at least one of the one or more other devices is within communication range.

In some embodiments, subscribe may be used in active mode. For example, the patient device may activate subscribe with the service name that represents the need of local medical help. In some embodiments, the active subscribe may be used and subscribe messages may be transmitted at a particular rate (e.g., as frequently as possible) with the objective to attract local helpers that publish correspondingly. Once enough and/or capable help has been identified, the patient device may be configured to discontinue the subscribe operations or, in some embodiments, transmit subscribe message less frequently.

In some embodiments, the service name and the corresponding Service ID field value may represent the generic need of local help, the Service Info field in the subscribe messages may be used to carry information about the situation and/or type of help. The subscribe messages may be generated to contain information that is helpful to determine in peer devices whether the person carrying the peer device may help with the situation. The Service Info field may, for example, provide information indicative of health conditions (e.g., whether the person asking for help has heart problems, has diabetes, or the like). In some embodiments, identification information (i.e. information configured to identify the person in need of help) may not be released at this point (e.g., in the help request transmission phase), but only once a helper is identified and determined to be within a predefined distance to the patient and, in some embodiments, is determined to need more information. This will be described in further detail below.

Alternatively, in some embodiments, a matching filter field may be used to contain a sequence of field values that determine the type of help needed. In some embodiments, this approach may be used only when necessary to attract a particular type of help (e.g., one trained in a specific discipline or the like) instead of attracting any help.

In some embodiments, follow-up messages may be used between the patient and/or patient device and a helper and/or helper device or potential helper and their device to obtain more information, such as information particular to the current situation or a condition of the patient.

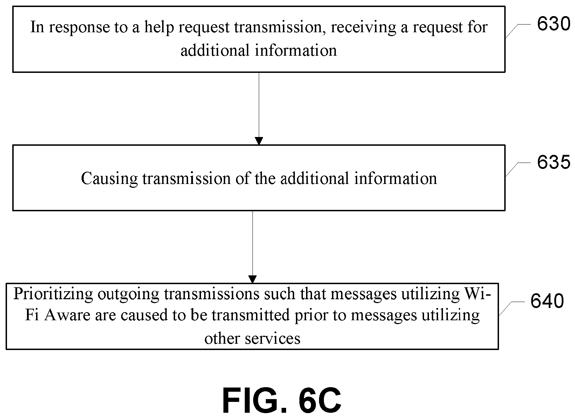

As such, as shown in block 630 of Figure 6C, an apparatus, such as apparatus 20 embodied by computing device 10 or the user device 16, may be configured to, in response to a help request transmission, receive a request for additional information. The apparatus may therefore include means, such as the processor 22, the communication interface 26 or the like, for in response to a help request transmission, receiving a request for additional information. As shown in block 635 of Figure 6C, an apparatus, such as apparatus 20 embodied by computing device 10 or the user device 16, may be configured to cause transmission of the additional information. The apparatus may therefore include means, such as the processor 22, the communication interface 26 or the like for causing transmission of the additional information.

In some embodiments, the use of follow-up messages may be utilized independent of whether the helper and/or helper device is within a predefined distance and is instead available to any helper device capable of receiving the help request transmission. In some embodiments, the follow up messages may be used to aid in a determination of whether the helper is capable of helping the patient.

In some embodiments, the determination of particular conditions identified in the condition detection process may be configured to initiate and/or operate the Wi-Fi Aware stack with high priority parameters, which prioritizes related messaging over other services. For example, a device may be configured to allow or enable transmission of subscribe messages every NAN Discovery Window and, in some embodiments, multiple times per window in the event a particular condition is identified. In some embodiments, a device may be configured to transmit subscribe messages upfront in the NAN Discovery Window to maximize probability of successful transmissions. Additionally or alternatively, a device may be configured to perform NAN scanning with increased frequency to maximize potential of discovery of all possible NAN Clusters and subsequently to maximize potential of discovery of possible local helpers.

As such, as shown in block 640 of Figure 6C, an apparatus, such as apparatus 20 embodied by computing device 10 or the user device 16, may be configured to cause prioritization of outgoing transmissions such that messages that relate to attraction of local help utilizing Wi-Fi Aware are caused to be transmitted prior to messages utilizing other services. The apparatus may therefore include means, such as the processor 22, the communication interface 26 or the like, for prioritizing outgoing transmissions such that messages that relate to attraction of local help utilizing Wi-Fi Aware are caused to be transmitted prior to messages utilizing other services.

In the case of using the Bluetooth LE, the computing device 10 may be configured to scan for user devices 16 (operated by a person willing and/or able to help) and once a viable user device 16 is found, request a connection to exchange more information. In some embodiments, connection may be essential because, for example, a scanning device (i.e. the computing device 10) may have no means to provide any information other than address in advertising channels. Thus a connection may be needed for the computing device to provide any information, for example, regarding the type of help needed. The computing device 10 may scan with high duty cycle to maximize discovery probability and minimize discovery time.

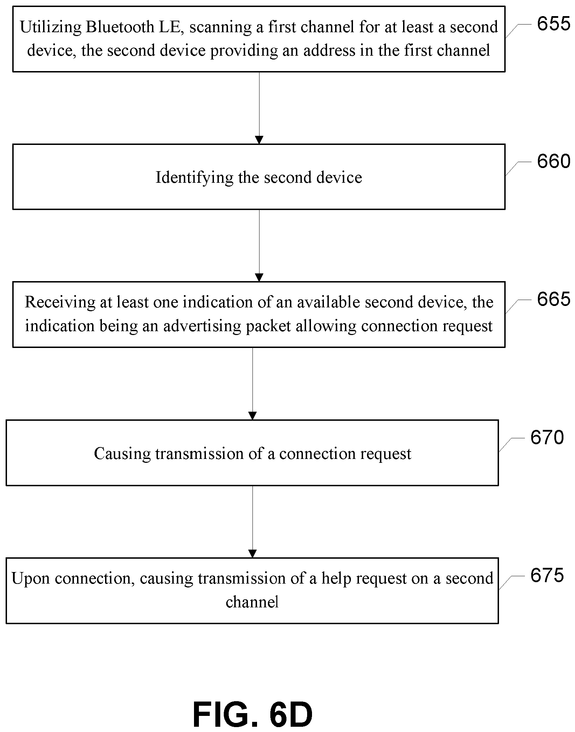

In some embodiments, the apparatus may be configured for scanning for other devices, and subsequently receiving an advertisement packet, which may result in the identification of a second device and it may result also in the identification of required help. In some embodiments, the advertising packets may comprise information indicative of need of help. Such information may be simply an indication of a service that may be used to attract and offer help locally. Alternatively the information may indicate also what type of help is needed or offered. In some embodiments, in those instances in which only the device id is available in the advertisement packet, a lot of connections may be formed during which one will recognize that there is no need for devices to interact. As such, as shown in block 655 of Figure 6D, an apparatus, such as apparatus 20 embodied by computing device 10 or the user device 16, may be configured to, utilizing Bluetooth LE, scan a first channel for at least a second device, the second device providing an address in the first channel. The apparatus may therefore include means, such as the processor 22, the communication interface 26 or the like, utilizing Bluetooth LE, scanning a first channel for at least a second device, the second device providing an address in the first channel.

As shown in block 660 of Figure 6D, an apparatus, such as apparatus 20 embodied by computing device 10 or the user device 16, may be configured to identify the second device. The apparatus may therefore include means, such as the processor 22, the communication interface 26 or the like for identifying the second device.

As shown in block 665 of Figure 6D, an apparatus, such as apparatus 20 embodied by computing device 10 or the user device 16, may be configured to receive at least one indication of an available second device, the indication being an advertising packet allowing connection request. The apparatus may therefore include means, such as the processor 22, the communication interface 26 or the like for receiving at least one indication of an available second device, the indication being an advertising packet allowing connection request.

As shown in block 670 of Figure 6D, an apparatus, such as apparatus 20 embodied by computing device 10 or the user device 16, may be configured to cause transmission of a connection request. The apparatus may therefore include means, such as the processor 22, the communication interface 26 or the like for causing transmission of a connection request.

In some embodiments, connection may be established, whereas in other embodiments, connection may not be established. In some embodiments, as shown in block 675 of Figure 6D, an apparatus, such as apparatus 20 embodied by computing device 10 or the user device 16, may be configured to, upon connection, cause transmission of a help request on a second channel. The apparatus may therefore include means, such as the processor 22, the communication interface 26 or the like for, upon connection causing transmission of a help request on a second channel. In those embodiments in which connection is not established, a second channel may be used for the help request transmission in the event that the first channel does not offer a relevant device for connection. As such the apparatus, such as apparatus 20 embodied by computing device 10 or the user device 16, may be configured to cause transmission on a second channel. The apparatus may therefore include means, such as the processor 22, the communication interface 26 or the like for causing transmission on a second channel. For example, the second channel may be used for the help request transmission in the event that the first channel does not offer a relevant device for connection.

Independent of the local wireless network providing the transmission and/or connection, once the help request transmission is sent, a helper device, for example, operated by a helper or potential helper (e.g., a person capable of providing medical help to the patient) may detect the presence of the help request transmission.

In one exemplary embodiment, a person who is willing and capable of offering medical help may possess and/or operate a device, such as a smart phone equipped with a local wireless interface (e.g., Wi-Fi Aware / NAN or BLE active). The local wireless interface may be active at times when the person is willing/capable/enforced/employed to provide local help. For example, the willingness or obligation to provide local help may originate from one's personal interests or attitude and/or a legal obligation related, for example, to one's occupation. As such, the helper device may require input indicative of the helper's willingness to help or, in some embodiments, may be configured to identify itself as being possessed or operated by a person willing and/or obligated to help. In either or both instances, once the helper device provides a response indicative of an affirmative reply to the help request transmission, the helper device may operate as follows.

When Wi-Fi Aware is used, the helper device may use the Wi-Fi Aware interface to publish. In some embodiments, the helper device may use unsolicited publish with the service name specified for the purpose as defined earlier. As noted earlier, the preferred approach is to have a service name specified for local help discovery without specifying the type of help in the service name itself. Thus whenever a person that subscribes for help is close by, the patient is discovered and the helper device or the helper may obtain more information on the situation.

When the BLE is used, the helper device may use the BLE interface to advertise. The advertising is power efficient and can be used for long periods of time without any significant effect on the device's battery lifetime. In some embodiments, the helper device may use connectable undirected advertising with the advertising packets containing information about offered help in the Advertising Data field.

### HELP IS ON THE WAY

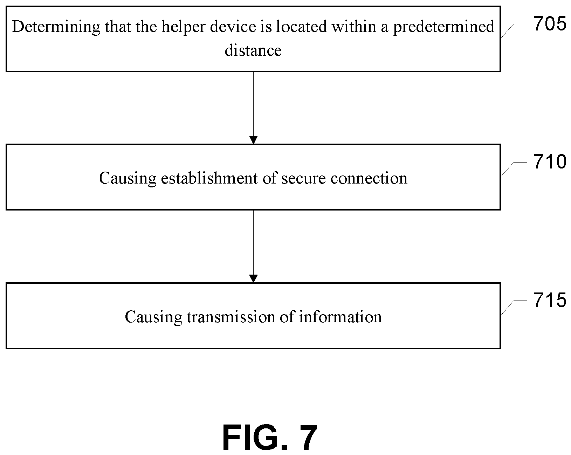

Once a helper device is identified, relevant information may be provided to the helper device. Figure 7 is an example flowchart illustrating a method for performing an information transfer process in accordance with an embodiment of the present invention. It should be appreciated that the operations of Figure 7 may be performed by a smart phone. In some embodiments, the operations of Figure 7 are not limited to any particular network (e.g., cellular systems). For example, non-cellular solutions such as a wireless local area network (WLAN) or a wireless personal area network (WPAN) (e.g., Wi-Fi Aware or over Bluetooth LE) may similarly permit the information transfer process.

In one exemplary embodiment, once a helper has discovered a person close by who needs help, the helper has confirmed that one can offer required help and the helper is close to the patient (e.g., within a few meters of the patient), the helper device and the patient device may establish a secured connection to exchange more information. The connection may be used, for example, to transfer relevant information (e.g., the reason(s) of the proximity health alert). In some embodiments, for example where a Wi-Fi Aware device is used, NAN ranging (a peer-to-peer Wi-Fi ranging) may be utilized to ensure that the connection is established only to a device close enough to the patient to ensure that the connection is established and information is transmitted to the right device.

As such, as shown in block 705 of Figure 7, an apparatus, such as apparatus 20 embodied by computing device 10 or the user device 16, may be configured to determine that the helper device is located within a predetermined distance. The apparatus may therefore include means, such as the processor 22, the communication interface 26 or the like, for determining that the helper device is located within a predetermined distance. As shown in block 710 of Figure 7, an apparatus, such as apparatus 20 embodied by computing device 10 or the user device 16, may be configured to cause establishment of secure connection. The apparatus may therefore include means, such as the processor 22, the communication interface 26 or the like for causing establishment of secure connection.

Once a secure connection is established, transmission of relevant information may begin. As such, as shown in block 715 of Figure 7, an apparatus, such as apparatus 20 embodied by computing device 10 or the user device 16, may be configured to cause transmission of information. The apparatus may therefore include means, such as the processor 22, the communication interface 26 or the like for causing transmission of information.

In some embodiments, the operations shown in Figure 7 may be utilized to provide any of at least the following information, either in the discovery phase or in a connection established between the patient device and the potential helper device: The detected symptom and the acuteness of the symptoms; The medical record of the person that likely is related to the symptom or to provide additional details that needs to be considered in the treatment; The measurement data of the patient; Person height and weight, blood type, etc. information; The information of the first aid need for the patient; The device may play a video of the required first aid; A URL that provides the information; A link to contact the 911 dispatcher or ambulance or hospital personnel that is handling the patient; Information does the patient already get first aid. For instance the measurement data or detecting other device in close proximity <1m distance may indicate that the patent is getting first aid; and the skill level of the persons giving the first aid may be reported. For instance, it may be noted if a doctor or a nurse is giving the first aid.

In some embodiments, the user device 16 may be configured to execute an application that sounds an alarm or the like. The user device 16 may be configured through the user profile settings. In some embodiments, user device 16 may be configured with a "do-not-disturb" mode in which only transmitted help requests indicative of only those conditions meeting particular criteria trigger the alarm (e.g., only the most severe alarms are played). Alternatively, user device 16 may be configured to play all alarms.

In some embodiments, density of the devices in area may affect which alarms are played. For example, in areas in which less than a predefined threshold of devices are available or identified, even minor alarms may be played, whereas in areas in which more than the predefined threshold of devices area available or more than a second predefined threshold of devices area available, only a subset or portion of conditions trigger alarms in a user device 16.

In some embodiments, a geolocation of the user device 16 may affect which alarms are played. In some embodiments, skills and/or occupation of the operator of user device 16 may affect which alarms are triggered. That is, some people may be specialized to specific topics, like heart attacks and as such, may receive all heart related alarms. In some embodiments, the application may consult or otherwise consider a calendar of the operator of user device 16. If the operator of user device 16 has, for example, a high priority meeting, particular, for example, low priority alarms may not played.

User device 16, or the application executed thereon, may be configured to use a specific ringtone, vibration and/or screen view to notify the operator of the alarm. User device 16 may provide an indication that it has received an alarm, or that the operator has reacted to the alarm and is responding (e.g., intending to provide the first aid to the patient). In some embodiments, if the computing device 10 detects that many devices (e.g., greater than a predefined threshold) are in close proximity (e.g., within a predefined distance), the computing device 10 may stop transmission of the proximity health alert and maintain the information sharing functionality with devices in close proximity.

### REQUESTING HELP

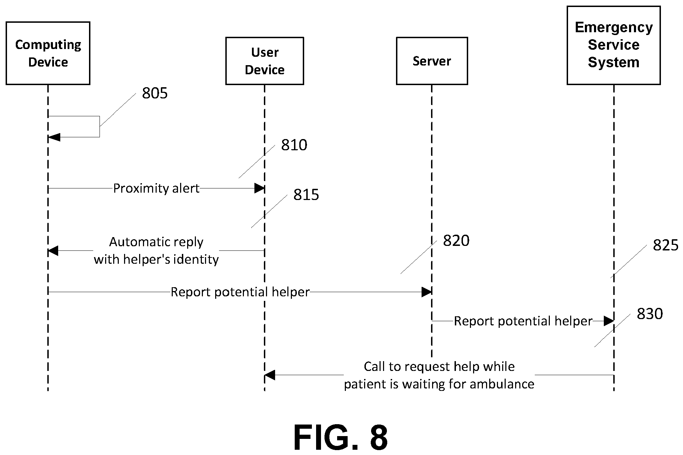

Figure 8 is an example flowchart illustrating a method for supplementing existing emergency service architecture with immediate local help identified via the proximity alert processes shown above, in accordance with an embodiment of the present invention.

As shown, in step 805, the condition detection process described above is performed. The proximity alert is then transmitted in block 810 and the reply is shown in block 815. Specifically, when a user device (e.g., a smart phone) operated by a potential helper (e.g., a person with medical skills) receives a proximity alert (e.g., via Wi-Fi Aware or Bluetooth LE advertisement), the user device may reply with a message identifying the operator of the user device (e.g., the potential helper). As shown in Block 820, the computing device 10 may then transmit a message indicating at least the identity and, in some embodiments, other relevant information to the server, the server being responsible for managing the patient's data or health care services or the like. The server may than transmit, as shown in Block 825, the data to an emergency center (e.g., 911), the patient's doctor or the like. As shown in Block 830, the emergency center or doctor may then then call the potential helper and request help as well as provide relevant information regarding the patient or the patient's condition. As such, a potential helper may be instantly or near instantly provided information specific to the patient or patient's condition.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A method for facilitating the transmission of a proximity health alert from a device to other local devices upon a determination that a person is in need of help and that local, immediate help is sought comprising:
performing a condition detection process (405) resulting in a determination (410) of whether help is needed;
causing activation (415) of a local wireless network interface in an instance in which the performance of the condition detection process results in a determination that local help is needed;
continuing to perform the condition detection process (405) in an instance in which performance of the condition detection process results in a determination that help is not needed; and
performing a help request transmission process (420), **characterised by** the help request transmission process comprising:
utilizing Bluetooth LE, scanning (655) a first channel for at least a second device, the second device providing an address in the first channel;
identifying (660) the second device;
receiving (665) at least one indication of an available second device, the indication being an advertising packet allowing connection request, wherein the advertising packet is indicative of at least one of a connectable undirected advertising or scannable undirected advertising;
causing transmission (670) of a connection request; and
upon connection, causing transmission (675) of a help request on a second channel.

2. The method according to Claim 1, wherein in an instance in which the help request transmission process does result in identification of a helper device, the method further comprising:
causing transmission (425) of additional information to the helper device.

3. The method according to Claim 1, wherein the condition detection process comprises:
causing reception (505) of sensor data from sensors;
causing transmission (510) of the sensor data to server; and
receiving (515), from the server, information indicative of the determination of whether help is needed.

4. The method according to any of Claim 1 or 2, wherein the condition detection process comprises:
causing reception of sensor data from sensors;
determining, via a processor, whether help is needed.

5. The method according to any of Claim 1 to 4, wherein the local wireless network interface is WLAN, Wi-Fi Aware, Bluetooth LE, or ZigBee.

6. The method according to any of Claims 1 to 5, wherein the help request transmission process comprises:
utilizing Wi-Fi Aware, identifying (605), on the basis of a geographic location, a service capable of transmitting a help request message;
generating (610) the help request message; and
causing transmission (615) of the help request message on the service.

7. The method according to any of Claims 1 to 5, wherein the help request transmission process comprises:
utilizing Wi-Fi Aware, identifying (605) a service capable of transmitting a help request message;
generating (610) the help request message; and
causing transmission (615) of the help request message on the service.

8. The method according to any of Claims 1 to 5, wherein the help request transmission process comprises:
utilizing Wi-Fi Aware, activating (620) a subscribe functionality to enable identification of one or more other devices located within communication range; and
while continuing to utilize Wi-Fi Aware, receiving (625) one or more publish notifications from at least one of the one or more other devices indicating that the at least one of the one or more other devices is within communication range.

9. The method according to any of Claims 1 to 4, wherein the help request transmission process comprises:
in response to a help request transmission, receiving (630) a request for additional information; and
causing transmission (635) of the additional information.

10. The method according to any of Claims 1 to 4, wherein the help request transmission process comprises:
prioritizing (640) outgoing transmissions such that messages utilizing Wi-Fi Aware are caused to be transmitted prior to messages utilizing other services.

11. The method according to any of Claims 1 to 10, wherein the help request transmission process comprises:
determining (705) that the helper device is located within a predetermined distance; and
causing (710) establishment of secure connection.

12. An apparatus configured to perform the method as claimed in any of claims 1 to 11.

13. A computer program configured to cause a method according to at least one of claims 1 to 11 to be performed.

## Patentansprüche

1. Verfahren zum Ermöglichen der Übertragung einer Näherungsgesundheitswarnung von einer Vorrichtung zu anderen lokalen Vorrichtungen nach einer Bestimmung, dass eine Person Hilfe benötigt und dass lokale sofortige Hilfe gesucht wird, das Folgendes umfasst:
Durchführen eines Zustandsdetektionsprozesses (405), der in einer Bestimmung (410), ob Hilfe benötigt wird, resultiert;
Bewirken der Aktivierung (415) einer lokalen drahtlosen Netzwerkschnittstelle in einem Fall, in dem das Durchführen des Zustandsdetektionsprozesses in einer Bestimmung, dass lokale Hilfe benötigt wird, resultiert;
Fortsetzen des Durchführens des Zustandsdetektionsprozesses (405) in einem Fall, in dem das Durchführen des Zustandsdetektionsprozesses in einer Bestimmung, dass keine Hilfe benötigt wird, resultiert; und
Durchführen eines Hilfeanforderungsübertragungsprozesses (420), **dadurch gekennzeichnet, dass** der Hilfeanforderungsübertragungsprozess Folgendes umfasst:
Scannen (655) eines ersten Kanals auf mindestens eine zweite Vorrichtung unter Verwendung von Bluetooth LE, wobei die zweite Vorrichtung eine Adresse im ersten Kanal bereitstellt;
Identifizieren (660) der zweiten Vorrichtung;
Empfangen (665) von mindestens einer Anzeige einer verfügbaren zweiten Vorrichtung, wobei die Anzeige ein Ankündigungspaket ist, das eine Verbindungsanforderung erlaubt, wobei das Ankündigungspaket mindestens eines von einer ungerichteten verbindbaren Ankündigung oder einer ungerichteten scanbaren Ankündigung anzeigt;
Bewirken der Übertragung (670) einer Verbindungsanforderung; und
nach der Verbindung Bewirken der Übertragung (675) einer Hilfeanforderung auf einem zweiten Kanal.

2. Verfahren nach Anspruch 1, wobei das Verfahren in einem Fall, in dem der Hilfeanforderungsübertragungsprozess in der Identifikation einer Helfervorrichtung resultiert, ferner Folgendes umfasst:
Bewirken der Übertragung (425) von zusätzlichen Informationen zur Helfervorrichtung.

3. Verfahren nach Anspruch 1, wobei der Zustandsdetektionsprozess Folgendes umfasst:
Bewirken des Empfangs (505) von Sensordaten von Sensoren;
Bewirken der Übertragung (510) der Sensordaten zum Server; und
Empfangen (515) von Informationen, die die Bestimmung, ob Hilfe benötigt wird, anzeigen, vom Server.

4. Verfahren nach einem der Ansprüche 1 oder 2, wobei der Zustandsdetektionsprozess Folgendes umfasst:
Bewirken des Empfangs von Sensordaten von Sensoren;
Bestimmen via einen Prozessor, ob Hilfe benötigt wird.

5. Verfahren nach einem der Ansprüche 1 bis 4, wobei die lokale drahtlose Netzwerkschnittstelle WLAN, Wi-Fi Aware, Bluetooth LE oder ZigBee ist.

6. Verfahren nach einem der Ansprüche 1 bis 5, wobei der Hilfeanforderungsübertragungsprozess Folgendes umfasst:
Identifizieren (605) eines Dienstes, der in der Lage ist, eine Hilfeanforderungsnachricht zu übertragen, unter Verwendung von Wi-Fi Aware auf Basis eines geografischen Standorts;
Erzeugen (610) der Hilfeanforderungsnachricht; und
Bewirken der Übertragung (615) der Hilfeanforderungsnachricht auf dem Dienst.

7. Verfahren nach einem der Ansprüche 1 bis 5, wobei der Hilfeanforderungsübertragungsprozess Folgendes umfasst:
Identifizieren (605) eines Dienstes, der in der Lage ist, eine Hilfeanforderungsnachricht zu übertragen, unter Verwendung von Wi-Fi Aware;
Erzeugen (610) der Hilfeanforderungsnachricht; und
Bewirken der Übertragung (615) der Hilfeanforderungsnachricht auf dem Dienst.

8. Verfahren nach einem der Ansprüche 1 bis 5, wobei der Hilfeanforderungsübertragungsprozess Folgendes umfasst:
Aktivieren (620) einer Teilnahmefunktionalität, um die Identifikation von einer oder mehreren anderen Vorrichtungen, die sich im Kommunikationsbereich befinden, zu ermöglichen, unter Verwendung von Wi-Fi Aware; und
Empfangen (625) von einer oder mehreren Veröffentlichungsbenachrichtigungen von mindestens einer der einen oder der mehreren anderen Vorrichtungen, die anzeigen, dass sich die mindestens eine der einen oder der mehreren anderen Vorrichtungen im Kommunikationsbereich befindet, während der fortgesetzten Verwendung Wi-Fi Aware.

9. Verfahren nach einem der Ansprüche 1 bis 4, wobei der Hilfeanforderungsübertragungsprozess Folgendes umfasst:
Empfangen (630) einer Anforderung von zusätzlichen Informationen in Reaktion auf eine Hilfeanforderungsübertragung; und
Bewirken der Übertragung (635) der zusätzlichen Informationen.

10. Verfahren nach einem der Ansprüche 1 bis 4, wobei der Hilfeanforderungsübertragungsprozess Folgendes umfasst:
Priorisieren (640) von abgehenden Übertragungen, derart, dass bewirkt wird, dass Nachrichten, die Wi-Fi Aware verwenden, vor Nachrichten übertragen werden, die andere Dienste verwenden.

11. Verfahren nach einem der Ansprüche 1 bis 10, wobei der Hilfeanforderungsübertragungsprozess Folgendes umfasst:
Bestimmen (705), dass sich die Helfervorrichtung in einem vorbestimmten Abstand befindet; und
Bewirken (710) des Aufbaus einer sicheren Verbindung.

12. Einrichtung, die dazu ausgelegt ist, das Verfahren wie in einem der Ansprüche 1 bis 11 beansprucht durchzuführen.

13. Computerprogramm, das dazu ausgelegt ist zu bewirken, dass ein Verfahren nach mindestens einem der Ansprüche 1 bis 11 durchgeführt wird.

## Revendications

1. Procédé pour faciliter la transmission d'une alerte de santé de proximité d'un dispositif à d'autres dispositifs locaux lors d'une détermination qu'une personne a besoin d'aide et qu'une aide locale immédiate est recherchée comprenant :
l'exécution d'un processus de détection de condition (405) aboutissant à une détermination (410) de si une aide est nécessaire ;
le fait de provoquer l'activation (415) d'une interface de réseau sans fil local dans une circonstance dans laquelle l'exécution du processus de détection de condition aboutit à une détermination qu'une aide locale est nécessaire ;
le fait de continuer à exécuter le processus de détection de condition (405) dans une circonstance dans laquelle l'exécution du processus de détection de condition aboutit à une détermination qu'une aide n'est pas nécessaire ; et
l'exécution d'un processus de transmission de demande d'aide (420), **caractérisé par** le processus de transmission de demande d'aide comprenant :
en utilisant Bluetooth LE, le balayage (655) d'un premier canal pour au moins un second dispositif, le second dispositif fournissant une adresse dans le premier canal ;
l'identification (660) du second dispositif ;
la réception (665) d'au moins une indication d'un second dispositif disponible, l'indication étant un paquet publicitaire autorisant une demande de connexion, dans lequel le paquet publicitaire est indicatif d'au moins l'une d'une publicité non dirigée connectable ou d'une publicité non dirigée scannable ;
le fait de provoquer la transmission (670) d'une demande de connexion ; et
lors de la connexion, le fait de provoquer la transmission (675) d'une demande d'aide sur un second canal.

2. Procédé selon la revendication 1, dans lequel dans une circonstance dans laquelle le processus de transmission de demande d'aide aboutit à l'identification d'un dispositif d'aidant, le procédé comprenant en outre :
le fait de provoquer la transmission (425) d'informations additionnelles au dispositif d'aidant.

3. Procédé selon la revendication 1, dans lequel le processus de détection de condition comprend :
le fait de provoquer la réception (505) de données de capteur en provenance de capteurs ;
le fait de provoquer la transmission (510) des données du capteur à un serveur ; et
la réception (515), en provenance du serveur, d'informations indicatives de si une aide est nécessaire.

4. Procédé selon l'une quelconque des revendications 1 ou 2, dans lequel le processus de détection de condition comprend :
le fait de provoquer la réception de données de capteur en provenance de capteurs ;
la détermination, via un processeur, de si une aide est nécessaire.

5. Procédé selon l'une quelconque des revendications 1 à 4, dans lequel l'interface de réseau sans fil local est WLAN, Wi-Fi Aware, Bluetooth LE, ou ZigBee.

6. Procédé selon l'une quelconque des revendications 1 à 5, dans lequel le processus de transmission de demande d'aide comprend :
en utilisant Wi-Fi Aware, l'identification (605), sur la base d'un emplacement géographique, d'un service capable de transmettre un message de demande d'aide ;
la génération (610) du message de demande d'aide ; et
le fait de provoquer la transmission (615) du message de demande d'aide sur le service.

7. Procédé selon l'une quelconque des revendications 1 à 5, dans lequel le processus de transmission de demande d'aide comprend :
en utilisant Wi-Fi Aware, l'identification (605) d'un service capable de transmettre un message de demande d'aide ;
la génération (610) du message de demande d'aide ; et
le fait de provoquer la transmission (615) du message de demande d'aide sur le service.

8. Procédé selon l'une quelconque des revendications 1 à 5, dans lequel le processus de transmission de demande d'aide comprend :
en utilisant Wi-Fi Aware, l'activation (620) d'une fonctionnalité d'abonnement pour permettre l'identification d'un ou plusieurs autres dispositifs situés à portée de communication ; et
tout en continuant à utiliser Wi-Fi Aware, la réception (625) d'une ou plusieurs notifications de publication en provenance d'au moins un des un ou plusieurs autres dispositifs indiquant que le au moins un des un ou plusieurs autres dispositifs est à portée de communication.

9. Procédé selon l'une quelconque des revendications 1 à 4, dans lequel le processus de transmission de demande d'aide comprend :
en réponse à une transmission de demande d'aide, la réception (630) d'une demande pour des informations additionnelles ; et
le fait de provoquer la transmission (635) des informations additionnelles.

10. Procédé selon l'une quelconque des revendications 1 à 4, dans lequel le processus de transmission de demande d'aide comprend :
la priorisation (640) de transmissions sortantes de sorte que des messages utilisant Wi-Fi Aware soient amenés à être transmis avant des messages utilisant d'autres services.

11. Procédé selon l'une quelconque des revendications 1 à 10, dans lequel le processus de transmission de demande d'aide comprend :
la détermination (705) que le dispositif d'aidant est situé à une distance prédéterminée ; et
le fait de provoquer (710) l'établissement d'une connexion sécurisée.

12. Appareil configuré pour exécuter le procédé selon l'une quelconque des revendications 1 à 11.

13. Programme informatique configuré pour amener un procédé selon au moins une des revendications 1 à 11 à être exécuté.
